Europäisches Patentamt

European Patent Office

Office européen des brevets

(19)

(11) Numéro de publication: **0 362 037**
**A2**

(12) **DEMANDE DE BREVET EUROPEEN**

(21) Numéro de dépôt: 89402611.1

(22) Date de dépôt: 25.09.89

(51) Int. Cl.5: **C07C 45/62 , C07C 47/02 , C07C 47/228**

(30) Priorité: 26.09.88 FR 8812521

(43) Date de publication de la demande:
04.04.90 Bulletin 90/14

(84) Etats contractants désignés:
AT BE CH DE ES FR GB GR IT LI LU NL SE

(71) Demandeur: RHONE-POULENC SANTE
20, avenue Raymond Aron
F-92160 Antony(FR)

(72) Inventeur: Grosselin, Jean-Michel
15 rue Terraille
F-69001 Lyon(FR)

(74) Mandataire: Le Pennec, Magali et al
RHONE-POULENC SANTE Service Brevets
Santé 25 Quai Paul Doumer
F-92408 Courbevoie Cédex(FR)

(54) Procédé de préparation d'aldéhydes saturés par hydrogénation d'aldéhydes alfa, beta-insaturés.

(57) Procédé de préparation d'aldéhydes saturés par hydrogénation d'aldéhydes $\alpha,\beta$-insaturés en milieu biphasique en présence d'un catalyseur constitué d'un dérivé du rhodium associé à un ligand soluble dans l'eau ou d'un complexe du rhodium avec un ligand soluble dans l'eau.

EP 0 362 037 A2

# PROCEDE DE PREPARATION D'ALDEHYDES SATURES PAR HYDROGENATION D'ALDEHYDES α,β-INSATURES

La présente invention concerne un procédé de prépration d'aldéhydes saturés par hydrogénation des aldéhydes α,β-insaturés en milieu biphasique.

Il est connu, d'après F.H. Jardine et G. Wilkinson, J. Chem. Soc (C), 270 (1967) de préparer des aldéhydes saturés par hydrogénation d'aldéhydes insaturés en utilisant du tris(triphénylphosphine) chlororhodium. Cependant pour favoriser l'hydrogénation aux dépens de la décarbonylation, il est nécessaire d'employer des solutions diluées de l'aldéhyde insaturé ce qui rend difficile l'isolement du produit de réaction ou bien d'opérer sous forte pression d'hydrogène ce qui conduit à la formation d'une quantité importante d'alcool saturé correspondant.

Il est connu également, d'après C. Larpent et coll., Tetrahedron Letters, 28 (22) 2507 (1987), d'hydrogéner les oléfines en milieu biphasique aqueux en utilisant comme catalyseur des complexes du rhodium avec des ligands hydrosolubles tels que la tri(métasulfophényl) phosphine (TPPTS), les oléfines pouvant être fonctionnalisées par des groupements difficilement réductibles tels que des fonctions cétones ou acides.

Il a maintenant été trouvé, et c'est ce qui fait l'objet de la présente invention, que les aldéhydes α,β-insaturés peuvent être hydrogénés sélectivement en aldéhydes saturés en opérant dans un milieu biphasique constitué d'une phase organique et d'une phase aqueuse non miscible en présence d'un catalyseur constitué d'un dérivé du rhodium associé à un ligand hydrosoluble ou d'un complexe du rhodium avec un ligand hydrosoluble à une température comprise entre 0 et 100° C et sous une pression d'hydrogène comprise entre 1 et 100 bars.

Les ligands hydrosolubles sont plus particulièrement les phosphines hydrosolubles qui sont décrites dans le brevet français FR 76 22824 (2 366 237) et plus spécialement la tri(métasulfophényl) phosphine (TPPTS).

Les dérivés du rhodium qui conviennent particulièrement bien sont choisis parmi les sels inorganiques ou organiques et les complexes du rhodium comme par exemple RhCl₃, RhBr₃, Rh₂O, Rh₂O₃, Rh(NO₃)₃, Rh(CH₃COO)₃, Rh(CH₃COCHCOCH₃)₃, [RhCl (cyclooctadiène)1,5)]₂, [RhCl(CO)₂]₂ ou RhCl₃(C₂H₅NH₂)₃. Il est avantageux d'utiliser [RhCl (cyclooctadiène-1,5)]₂.

Généralement, la réaction est effectuée dans l'eau, l'aldéhyde α,β-insaturé et l'aldéhyde saturé constituant la phase organique. Il est possible cependant de mettre en oeuvre le procédé selon l'invention en présence d'un solvant organique utilisé en quantité telle que le mélange réactionnel est constitué d'une phase organique et d'une phase hydroorganique non miscible.

Les solvants organiques qui conviennent particulièrement bien sont choisis parmi les solvants non miscibles ou peu miscibles à l'eau. Plus particulièrement, les solvants sont choisis parmi les alcools (isopropanol), les éthers (éther éthylique, tertiobutyléther), les cétones (méthylisobutylcétone), les esters (acétate de méthyle, acétate d'éthyle, acétate de butyle) et les hydrocarbures aliphatiques ou aromatiques éventuellement halogénés (hexane, toluène, chlorure de méthylène, chloroforme, chlorobenzène) pris seuls ou en mélange.

L'hydrogénation peut être réalisée à une température comprise entre 0 et 200° C mais de préférence entre 10 et 100° C.

L'hydrogénation peut être réalisée sous une pression de 1 à 100 bars mais de préférence entre 10 et 50 bars.

Généralement, on utilise de 0,00 à 0,05 mole de dérivé du rhodium par mole d'aldéhyde α,β-insaturé mis en oeuvre, de préférence 0,01.

Généralement, on utilise 1 à 100 moles de ligand par rapport au rhodium.

Le catalyseur ou le système catalytique étant soluble dans l'eau, il peut être facilement séparé, en fin de réaction, par décantation et il peut être ainsi recyclé.

Le procédé selon l'invention permet d'obtenir les aldéhydes saturés avec une sélectivité généralement supérieure à 90 %.

Le procédé selon l'invention permet de préparer des aldéhydes aliphatiques ou araliphatiques saturés à partir d'aldéhydes aliphatiques ou araliphatiques α,β-insaturés éventuellement poly-insaturés.

Les exemples suivants, donnés à titre non limitatif, montrent comment l'invention peut être mise en pratique.

## EXEMPLE 1

Dans une ampoule de verre de 25 cm3, on introduit successivement $5 \times 10^{-4}$ atome-gramme de rhodium sous forme [RhCl (cyclooctadiène-1,5)-]₂, $2 \times 10^{-3}$ mole de TPPTS, 5 cm3 d'hexane, 5 cm3 d'eau et $24 \times 10^{-3}$ mole de crotonaldéhyde.

L'ampoule est introduite dans un autoclave de 125 cm3 qui est placé dans une enceinte permettant une agitation par secousses. Le système est purgé avec de l'hydrogène avant d'établir la pression à 20 bars. On fixe la température à 30°C.

Après 20 minutes, l'analyse du mélange réactionnel par chromatographie en phase gazeuse montre que :
- le taux de transformation du crotonaldéhyde est de 88,7 %
- la répartition des produits d'hydrogénation est la suivante :
buténol : 1,3 %
butanol : 1,8 %
butanal : 85 %
- le rendement en butanal est de 95,6 % par rapport au crotonaldéhyde mis en oeuvre.

La phase organique est séparée par décantation puis on ajoute à la phase aqueuse 5 cm3 d'hexane et 24 x 10$^{-3}$ mole de crotonaldéhyde. En opérant dans les mêmes conditions de température et de pression que précédemment, l'analyse du mélange réactionnel après 20 minutes d'hydrogénation, montre que :
- le taux de transformation du crotonaldéhyde est de 94 %
- le rendement en butanal est de 97,5 % par rapport au crotonaldéhyde transformé.

EXEMPLE 2

Dans une ampoule de verre de 25 cm3, on introduit successivement 5 x 10$^{-4}$ atome-gramme de rhodium sous forme [RhCl (cyclooctadiène-1,5)]$_2$, 2 x 10$^{-3}$ mole de TPPTS puis 5 cm3 d'eau et 5 cm3 de toluène. On ajoute ensuite 2 x 10$^{-2}$ mole de butène-2 al. L'ampoule est introduite dans un autoclave de 125 cm3 agité par secousses.

Le système est purgé à l'hydrogène. Après avoir fixé la pression d'hydrogène à 20 bars et la température à 40°C, on effectue l'hydrogénation pendant 20 minutes.

Le mélange réactionnel est analysé par chromatographie en phase gazeuse.

On obtient ainsi le butanal avec un rendement de 96 % par rapport au butène-2 al transformé.

EXEMPLE 3

On opère comme dans l'exemple 2 en remplaçant le butène-2 al par 2 x 10$^{-2}$ mole de méthyl-3 butène-2 al et en effectuant l'hydrogénation sous une pression de 20 bars à 80°C.

On obtient ainsi le méthyl-3 butanal avec un rendement de 96 % par rapport au méthyl-3 butène-2 al transformé.

EXEMPLE 4

On opère comme dans l'exemple 2 en remplaçant le butène-2 al par 2 x 10$^{-2}$ mole de cinnamaldéhyde et en effectuant l'hydrogénation sous une pression de 20 bars à 80°C.

On obtient ainsi le phényl-3 propanal avec un rendement de 94 % par rapport au cinnamaldéhyde transformé.

EXEMPLE 5

On opère comme dans l'exemple 2 en remplaçant le butène-2 al par 2 x 10$^{-2}$ mole de citral et en effectuant l'hydrogénation sous une pression de 40 bars à 60°C.

On obtient ainsi le diméthyl-3,7 octanal avec un rendement de 96 % par rapport au citral transformé.

**Revendications**

1 - Procédé de préparation d'aldéhydes aliphatiques ou araliphatiques saturés par hydrogénation d'aldéhydes aliphatiques ou araliphatiques α,β-insaturés caractérisé en ce que l'hydrogénation est effectuée dans un milieu biphasique constitué d'une phase organique et d'une phase essentiellement aqueuse non miscible en présence d'un catalyseur à base de rhodium choisi parmi les dérivés du rhodium associés à un ligand hydrosoluble et les complexes du rhodium avec un ligand hydrosoluble.

2 - Procédé selon la revendication 1 caractérisé en ce que les dérivés du rhodium associés à un ligand sont choisis parmi les sels minéraux ou organiques et les complexes du rhodium.

3 - Procédé selon la revendication 2 caractérisé en ce que les dérivés du rhodium associés à un ligand hydrosoluble sont choisis parmi RhCl$_3$, RhBr$_3$, Rh$_2$O, Rh$_2$O$_3$, Rh(NO$_3$)$_3$, Rh(CH$_3$COO)$_3$, Rh(CH$_3$COCHCOCH$_3$)$_3$, [RhCl (cyclooctadiène)-1,5)]$_2$, [RhCl(CO)$_2$]$_2$ ou RhCl$_3$(C$_2$H$_5$NH$_2$)$_3$.

4 - Procédé selon l'une des revendications 1 à 4 caractérisé en ce que le ligand hydrosoluble est choisi parmi les phosphines solubles dans l'eau.

5 - Procédé selon la revendication 4 caractérisé en ce que les phosphines solubles dans l'eau sont choisies parmi les phénylphosphines sulfonées.

6 - Procédé selon la revendication 5 caractérisé en ce que la phénylphosphine sulfonée est la tri(métasulfophényl) phosphine.

7 - Procédé selon la revendication 1 caractérisé en ce que l'on opère en outre en présence d'un solvant organique en quantité suffisante pour maintenir l'existence d'une phase organique et d'une phase essentiellement aqueuse non miscible.

8 - Procédé selon la revendication 7 caractérisé en ce que le solvant organique est choisi parmi les alcools, les éthers, les cétones, les esters et les hydrocarbures aliphatiques ou aromatiques éventuellement halogénés utilisés seuls ou en mélange.

9 - Procédé selon l'une des revendications 1 à 8 caractérisé en ce que l'on opère à une température comprise entre 0 et 100°C.

10 - Procédé selon l'une des revendications 1 à 8 caractérisé en ce que l'on opère sous une pression comprise entre 1 et 100 bars.